# FASCICULE DE BREVET EUROPEEN

(11) **EP 0 749 750 B1**
(45) Date de publication et mention de la délivrance du brevet: **09.07.1997**
(21) Numéro de dépôt: 96401171.2
(22) Date de dépôt: 31.05.1996
(51) Int. Cl.: A61K 7/48, A61K 7/42

(54) **Composition pour protéger et/ou lutter contre les tâches et/ou le vieillissement de la peau, ses utilisations**
Zusammensetzung zum Schutz der Haut und/oder zur Bekämpfung von Hautflecken und/oder Hautalterung und ihre Verwendungen
Composition for whitening, antiageing and/or protection of the skin and uses thereof

(30) Priorité: 20.06.1995 FR 9507335
(43) Date de publication de la demande: 27.12.1996
(73) Titulaire: L'OREAL, 75008 Paris (FR)
(72) Inventeur: Grollier, Jean-François, 75006 Paris (FR); Cantin, Hervé, 91420 Morangis (FR); Gagnebien, Didier, 92320 Chatillon (FR)
(74) Mandataire: Lhoste, Catherine

(56) Documents cités:
- EP-A- 0 390 682
- FR-A- 2 577 805
- CHEMICAL ABSTRACTS, vol. 121, no. 12, 19 Septembre 1994 Columbus, Ohio, US; abstract no. 141250, A. YAMAMOTO: "Skin cosmetics containing hydroquinone glycosides for preventing UV damage and skin aging." XP002000574 & JP-A-06 157 268 (SHISEIDO CO. LTD.) 3 Juin 1994

## Description

L'invention se rapporte à une composition cosmétique et/ou dermatologique destinée à prévenir et/ou lutter contre les taches cutanées et/ou le vieillissement de la peau et/ou à protéger la peau notamment contre les rayons ultraviolets et/ou dépigmenter la peau. Cette composition peut être appliquée sur le visage, le corps et/ou les jambes d'êtres humains ainsi que sur les mains, voire sur le cuir chevelu.

L'invention se rapporte aussi à une utilisation de cette composition pour le traitement cosmétique de la peau, à l'utilisation de cette composition pour la préparation d'une crème destinée au traitement dermatologique de la peau et à un procédé de traitement cosmétique.

Au cours du temps, il apparaît différents signes sur la peau, très caractéristiques du vieillissement, se traduisant notamment par une modification de la structure et des fonctions cutanées. Ce vieillissement est de nature physiologique mais est également photoinduit, c'est-à-dire dû à une exposition répétée de la peau à la lumière solaire, notamment ultraviolette. L'action de cette lumière sur les constituants de la peau et sur le sébum sécrété par la peau entraîne en particulier la formation de radicaux libres oxygénés (espèces chimiques radicalaires hydrophiles comme O₂·⁻, OH· et espèces radicalaires lipophiles comme CH 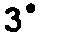 ) susceptibles de réagir avec les lipides de la peau et former des radicaux ROO·. Or, ces radicaux provoquent des dégâts importants, notamment dans les membranes cellulaires (perméabilité des membranes), les noyaux des cellules (mutation par action sur l'ARN ou ADN), et les tissus (nécroses, dégénérescence) : il est donc nécessaire de protéger la peau contre ces radicaux libres.

Les principaux signes cliniques du vieillissement cutané sont notamment l'apparition de ridules et de rides profondes, en augmentation avec l'âge ainsi qu'une désorganisation du "grain" de la peau : autrement dit, le micro-relief est moins régulier et présente un caractère anisotrope.

Par ailleurs, le teint de la peau est généralement modifié : il apparaît plus pâle et plus jaune ; ceci semble être dû essentiellement à une désorganisation de la microcirculation (moins d'hémoglobine au niveau du derme papillaire). De plus, de nombreuses taches colorées et/ou plus foncées apparaissent à la surface de la peau, et plus spécialement sur les mains, conférant à la peau une hétérogénéité. En général, ces taches sont dues à une production importante de mélanine dans l'épiderme et/ou le derme de la peau. Dans certains cas, ces taches peuvent devenir cancéreuses. Par ailleurs, il peut exister sur certaines zones de la peau des irritations diffuses et parfois des télangiectasies.

Un autre signe clinique du vieillissement est l'aspect sec et rêche de la peau qui est dû essentiellement à une desquamation plus importante : ces squames, en diffractant les rayons lumineux, participent aussi à l'aspect un peu gris du teint.

On remarque enfin une perte de fermeté et de tonicité de la peau qui, comme pour les rides et les ridules, s'explique du moins en partie par une atrophie dermique et épidermique ainsi qu'un aplatissement de la formation dermoépidermique ; la peau est moins épaisse et plus flasque, et l'épaisseur de l'épiderme diminue.

On constate donc que les signes cliniques du vieillissement cutané résultent essentiellement d'un dysfonctionnement des principaux mécanismes biologiques intervenant au niveau de la peau.

Aussi, la composition selon l'invention est une composition apte à prévenir et/ou lutter contre l'apparition du vieillissement et les signes de vieillissement, comme les rides, les ridules, à prévenir et/ou lutter contre les taches de pigmentation de la peau, quelle que soit leur origine, ainsi qu'à protéger la peau notamment par suppression de la formation de radicaux libres oxygénés.

Il existe sur le marché de nombreuses compositions destinées à lutter contre les taches cutanées, les signes du vieillissement et à protéger la peau des méfaits de la lumière solaire. Ces compositions présentent souvent une efficacité insuffisante.

L'actif le plus efficace, à ce jour, pour lutter contre les taches cutanées est l'hydroquinone. Malheureusement, ce composé est très instable et relativement toxique pour la peau, ce qui limite considérablement son utilisation. Aussi, on recherche de nouveau composé, voire de nouvelle association de composés, à propriété dépigmentante et/ou protectrice, ayant une efficacité comparable à l'hydroquinone mais ne présentant pas ses inconvénients.

L'invention a pour objet une nouvelle composition destinée à prévenir et/ou lutter contre le vieillissement de la peau et/ou les taches cutanées et/ou protéger la peau et/ou dépigmenter la peau, ayant des propriétés comparables, voire supérieures à celles de composition à base d'hydroquinone mais ne présentant pas ses inconvénients.

Selon une caractéristique essentielle de l'invention, cette composition à application topique contient dans un milieu cosmétiquement et/ou dermatologiquement acceptable de l'arbutine et au moins un filtre ultraviolet choisi dans le groupe constitué du benzylidène camphre et de ses dérivés, l'arbutine et le filtre étant en une quantité telle que cette association présente une synergie d'activité contre les radicaux libres. Le filtre peut être notamment un dérivé sulfoné ou sulfonaté du benzylidène camphre.

La présente invention a aussi pour objet une composition pour application topique, caractérisée en ce qu'elle contient dans un milieu cosmétiquement et/ou dermatologiquement acceptable, de l'arbutine et au moins un filtre ultraviolet choisi dans le groupe constitué des dérivés sulfonés ou sulfonatés du benzylidène camphre.

Ainsi, de façon surprenante, la demanderesse a découvert que l'utilisation simultanée d'arbutine et de benzylidène camphre et/ou l'un de ses dérivés permettait d'atténuer les rides et les ridules, de modifier le teint de la peau qui apparaît plus rosé, d'effacer les taches de pigmentation, de supprimer les squames et de donner une consistance plus élastique à la peau, tout en protégeant la peau contre les radicaux libres de l'oxygène.

Etant donné l'efficacité relativement faible de l'arbutine, il est tout-à-fait surprenant que le benzylidène camphre et /ou ses dérivés augmentent, de façon considérable, son efficacité antitache, antiradicalaire et protectrice notamment vis-à-vis des rayons ultraviolets. Par ailleurs, du fait que ces filtres ultraviolets ne présentent aucune propriété antiradicalaire, il est tout-à-fait surprenant que leur association avec l'arbutine confère à la composition des propriétés antiradicalaires et donc protectrices et qu'à certaines concentrations, et notamment à des concentrations de 0,05 à 0,15 % de chacun des composés, l'association d'arbutine et de filtre présente une synergie d'activité contre les radicaux libres.

Enfin, l'arbutine assure une augmentation de l'indice de protection du filtre UV assez surprenante du fait que l'arbutine n'est nullement un filtre.

Certes, le document FR2577805 décrit une composition de traitement de la peau à base de glucoside d'hydroquinone et susceptible de contenir un filtre. Toutefois, ce document ne décrit pas l'association particulière d'arbutine et de benzylidène camphre ou de dérivé de benzylidène camphre, et il n'enseigne pas que cette association puisse avoir des propriétés particulières.

L'arbutine est un dérivé glucosylé d'hydroquinone, l'hydroquinone-béta-D-glucopyranoside, généralement utilisée comme dépigmentant ; elle présente par rapport à l'hydroquinone une plus grande stabilité, facilitant son incorporation dans tous type de supports et assurant à ces derniers une plus longue durée d'utilisation. En outre, elle est beaucoup moins toxique que l'hydroquinone.

Les dérivés de benzylidène camphre utilisables dans l'invention sont, en particulier, les dérivés sulfonés et/ou sulfonatés ayant un large spectre d'absorption dans le domaine des UVA et UVB.

En particulier, les dérivés de benzylidène camphre utilisables dans l'invention présente la formule générale (a) suivante : 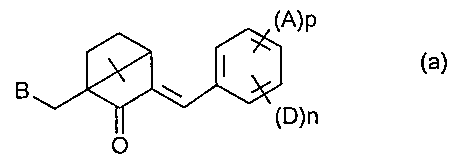 dans laquelle :
B représente -H ou -SO₃H,
0 ≤ p ≤ 1 avec B = -SO₃H quand p = 0,
0 ≤ n ≤ 4,
D représente un ou plusieurs radicaux alkyle ou alcoxy, identiques ou différents quand n ≥ 2, linéaires ou ramifiés contenant de 1 à 18 atomes de carbone environ, un radical halogéno, un radical hydroxyle.
A, de préférence en méta ou en para, représente
soit un radical SO₃H ;
soit un groupement : 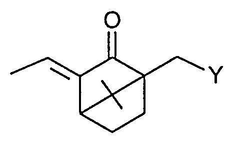 dans lequel Y représente H ou SO₃H ;
soit un groupement : 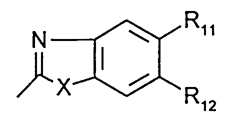 dans lequel :
R₁₁ désigne un atome d'hydrogène, un radical alkyle ou alcoxy, linéaire ou ramifié contenant de 1 à 6 atomes de carbone environ ou le radical -SO₃H, R₁₁ étant -SO₃H lorsque B = -H,
R₁₂ désigne un atome d'hydrogène, un radical alkyle ou alcoxy, linéaire ou ramifié, contenant de 1 à 6 atomes de carbone environ,
X est un atome d'oxygène, de soufre ou un groupement -NR-, R étant un atome d'hydrogène ou un radical alkyle, linéaire ou ramifié, contenant de 1 à 6 atomes de carbone environ,
et dans laquelle au moins une fonction -SO₃H est éventuellement neutralisée.

La neutralisation d'une ou plusieurs fonctions peut être obtenue à l'aide d'une base généralement utilisée dans le domaine cosmétique comme la soude, la triéthanolamine, la potasse.

On peut citer comme exemples particuliers de composés de formule (a) les dérivés de formules (I), (II), (III) suivantes :

### Formule (I) :

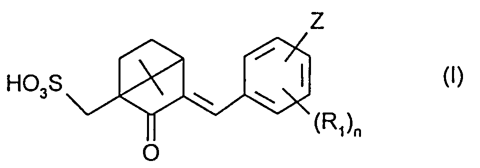

dans laquelle :
- Z, de préférence en position para ou méta, désigne un groupement 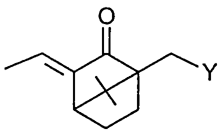 dans lequel Y représente -H ou -SO₃H, éventuellement neutralisé,
- n est égal à 0 ou est un nombre allant de 1 à 4 (0 ≤n≤ 4),
- R₁, représente un ou plusieurs radicaux alkyle ou alkoxy, identiques ou différents, linéaires ou ramifiés, contenant de 1 à 4 atomes de carbone environ.

Un composé de formule (I) particulièrement préféré est celui correspondant à n = 0 , Z en position para et Y = -SO₃H : l'acide benzène 1,4 [di(3-méthylidène-campho 10-sulfonique)], appelé aussi (selon la nomenclature CTFA - 5ème édition) l'acide téréphtalylidène - di-camphosulfonique.

### Formule (II) :

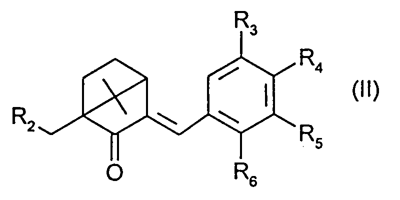

dans laquelle :
- R₂ désigne un atome d'hydrogène ou un radical -SO₃H,
- R₃, R_{4,} R₅ et R₆, identiques ou différents, représentent un groupement hydroxyle, un radical alkyle ayant de 1 à 4 atomes de carbone environ, linéaire ou ramifié, un radical alcényle ayant de 2 à 4 atomes de carbone environ, linéaire ou ramifié, un radical alcoxy ayant de 1 à 4 atomes de carbone, linéaire ou ramifié, un radical alcényloxy ayant de 2 à 4 atomes de carbone, linéaire ou ramifié, un radical halogéno ; de plus un radical R₃ à R₆ seulement peut être un radical -SO₃H, au moins un des radicaux R₃ à R₆ désignant le radical -SO₃H quand R₂ est un atome d'hydrogène. Une ou plusieurs fonctions -SO₃H peuvent aussi être neutralisées.

On peut citer comme exemples particuliers les composés suivants de formule (II) dans laquelle :
- R₄ désigne le radical -SO₃H en position para du benzylidènecamphre et R₂, R₃, R₅ et R₆ désignent chacun un atome d'hydrogène, c'est-à-dire l'acide 4'-sulfo 3-benzylidènecamphre.
- R₃, R₄, R₅ et R₆ désignent chacun un atome d'hydrogène et R₂ désigne un radical -SO₃H, c'est-à-dire l'acide 3-benzylidène campho-10 sulfonique.
- R₄ désigne un radical méthyle en position para du benzylidènecamphre, R₅ un radical -SO₃H et R₂, R₃ et R₆ représentent un atome d'hydrogène, c'est-à-dire l'acide 4'-méthyl 3'-sulfo 3-benzylidènecamphre.
- R₄ désigne un atome de chlore en position para du benzylidènecamphre, R₅ un radical -SO₃H et R₂, R₃ et R₆ représentent un atome d'hydrogène, c'est-à-dire l'acide 4'-chloro 3'-sulfo 3-benzylidènecamphre.
- R₄ désigne un radical méthyle en position para du benzylidènecamphre, R₃, R₅ et R₆ désignent un atome d'hydrogène et R₂ désigne un radical -SO₃H, c'est-à-dire l'acide 4'-méthyl 3-benzylidène campho 10-sulfonique.
- R₂ représente un radical -SO₃H, R₃ est un radical méthyle, R₄ un atome d'hydrogène, R₅ un radical tertiobutyle, R₆ un radical hydroxyle, c'est-à-dire l'acide (3-t-butyl 2-hydroxy 5-méthyl) 3-benzylidène campho-10-sulfonique.
- R₂ représente un radical -SO₃H, R₃ est un radical méthoxy, R₄ un atome d'hydrogène, R₅ un radical tertiobutyle, R₆ un radical hydroxyle, c'est-à-dire l'acide (3-t-butyl 2-hydroxy 5-méthoxy) 3-benzylidène campho-10-sulfonique.
- R₂ représente un radical -SO₃H, R₃ et R₅ désignent chacun un radical tertiobutyle, R₄ un radical hydroxyle, R₆ un atome d'hydrogène, c'est-à-dire l'acide (3,5-diterbutyl 4-hydroxy) 3-benzylidène campho-10-sulfonique.
- R₄ représente un radical méthoxy en para, R₅ représente -SO₃H, les radicaux R₂, R₃ et R₆ représentent H, c'est-à-dire l'acide 4'-méthoxy 3'-sulfo-3-benzylidène camphre.
- R₂ désigne un radical -SO₃H, R₃ et R₆ représentent H, R₄ et R₅ formant un radical méthylènedioxy, c'est-à-dire l'acide 3-(4,5-méthylènedioxy) benzylidène campho-10-sulfonique.
- R₂ représente un radical -SO₃H, R₄ un radical méthoxy et les radicaux R₃, R₅ et R₆ représentent H, c'est-à-dire l'acide 3-(4-méthoxy) benzylidène campho-10-sulfonique.
- R₂ représente un radical -SO₃H, R₄ et R₅ sont tous deux un radical méthoxy et les radicaux R₃ et R₆ représentent H, c'est-à-dire l'acide 3-(4,5-diméthoxy) benzylidène campho-10-sulfonique.
- R₂ représente un radical -SO₃H, R₄ est un radical n-butoxy et les radicaux R₃, R₅ et R₆ représentent un atome d'hydrogène, c'est-à-dire l'acide 3-(4-n.butoxy) benzylidène campho-10-sulfonique.
- R₂ représente un radical -SO₃H, R₄ est un radical n-butoxy, R₅ est un radical méthoxy et R₃ et R₆ désignent tous deux un atome d'hydrogène, c'est-à-dire l'acide 3-(4-n.butoxy 5-méthoxy) benzylidène campho-10-sulfonique.

### Formule (III) :

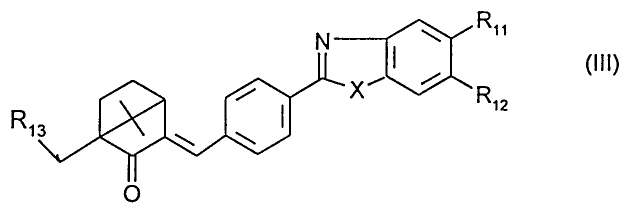

dans laquelle :
- R₁₁ désigne un atome d'hydrogène, un radical alkyle ou alcoxy, linéaire ou ramifié, contenant de 1 à 6 atomes de carbone environ ou un radical - SO₃H,
- R₁₂ désigne un atome d'hydrogène, un radical alkyle ou alcoxy, linéaire ou ramifié, contenant de 1 à 6 atomes de carbone environ,
- R₁₃ désigne un atome d'hydrogène ou un radical -SO₃H,
- l'un au moins des radicaux R₁₁ et R₁₃ désignant un radical -SO₃H,
- X est un atome d'oxygène ou de soufre ou un groupement -NR-, R étant un atome d'hydrogène ou un radical alkyle, linéaire ou ramifié, contenant de 1 à 6 atomes de carbone environ.

On peut citer comme exemple particulier de composé de formule (III) : le composé dans lequel X désigne un radical -NH-, R₁₁ désigne un radical -SO₃H, R₁₂ et R₁₃ désignent tous deux un atome d'hydrogène, c'est-à-dire l'acide 2-[4-(camphométhylidène) phényl] benzimidazole-5-sulfonique.

Les composés de structures (I), (II), (III) sont décrits dans le brevet US 4 585 597 et les brevets FR 2 236 515, 2 282 426, 2 645 148, 2 430 938, 2 592 380.

On peut citer comme autres exemples de dérivés du benzylidène camphre utilisables dans l'invention les composés de formule générale (b) suivante : 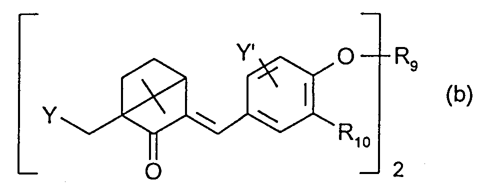 dans laquelle :
- R₉ désigne un radical divalent : -(CH₂)ₘ- ou -CH₂ -CHOH-CH₂-, m étant un nombre entier allant de 1 à 10 (1 ≤ m ≤ 10),
- R₁₀ désigne un atome d'hydrogène, un radical alcoxy contenant de 1 à 4 atomes de carbone environ ou un radical divalent - O - relié au radical R₉ lorsque celui-ci est divalent lui aussi,
- Y et Y' désignent un atome d'hydrogène ou un radical -SO₃H, au moins un de ces radicaux Y ou Y' est différent de l'hydrogène. Là encore la fonction - SO₃H peut être neutralisée.

On peut citer comme exemples particuliers les composés suivants de formule (b) dans laquelle Y représente -SO₃H, Y' est -H, R₁₀ est H et R₉ est -CH₂-CH₂-, c'est à dire l'acide éthylène bis [(4'-oxy benzylidène) 3-campho-10 sulfonique].

Selon l'invention, la quantité d'arbutine est celle classiquement utilisée dans le domaine cosmétique ou dermatologique. A titre d'exemple, on peut l'utiliser à raison de 0,05 % à 10 % en poids par rapport au poids total de la composition et de préférence de 0,5 % à 5 %.

De même, la quantité de filtre UV utilisable dans l'invention est celle généralement utilisée dans les domaines concernés. En pratique, on utilise de 0,1 % à 10 % en poids de filtre par rapport au poids total de la composition et de préférence de 0,1 % à 5 %.

La composition de l'invention peut présenter toutes les formes galéniques normalement utilisées pour une application topique telles que les solutions, les gels aqueux ou hydroalcooliques, les émulsions huile-dans-eau ou eau-dans-huile, et plus particulièrement les gouttelettes d'huile dispersées par des sphérules dans une phase aqueuse. Ces sphérules peuvent être des nanoparticules polymériques telles que les nanosphères et les nanocapsules ou mieux des vésicules lipidiques, les lipides de ces vésicules pouvant être ioniques ou non. La composition de l'invention peut se présenter sous forme d'une crème, d'une pommade, d'une lotion ou d'un sérum.

Les huiles utilisables dans l'invention sont celles généralement utilisées dans les domaines concernés. Elles peuvent être végétales, minérales ou synthétiques, éventuellement siliconées et/ou fluorées.

L'invention peut également contenir des adjuvants hydrophiles ou lipophiles comme des gélifiants, des conservateurs, des opacifiants, des émulsionnants, des coémulsionnants, des neutralisants, des parfums et leurs solubilisants ou peptisants, des matières colorantes comme les colorants et les pigments, des charges, ainsi que des actifs lipophiles ou hydrophiles autres que l'arbutine, le benzylidène camphre et l'un de ses dérivés.

Les quantités d'huile et d'eau sont généralement celles utilisées dans les domaines considérés et sont fonction de la forme galénique de la composition. Pour une émulsion huile-dans-eau ou une dispersion d'huile dans de l'eau par des sphérules lipidiques, l'huile peut représenter de 2 % à 40 % en poids par rapport au poids total de la composition.

De même les adjuvants sont utilisés en quantité habituelle et peuvent représenter, au total, de 0,1 % à 20 % en poids. Leur quantité est fonction de leur nature.

La composition de l'invention peut être appliquée par voie topique sur toutes les parties du corps et du visage, y compris sur le cuir chevelu, les jambes et les mains.

L'invention a aussi pour objet une utilisation de la composition définie ci-dessus pour le traitement cosmétique des rides et/ou les ridules de la peau ainsi qu'une utilisation de cette composition pour tonifier, protéger, hydrater et/ou raffermir la peau.

L'invention a encore pour objet une utilisation de la composition définie ci-dessus pour dépigmenter la peau et/ou traiter cosmétiquement les taches cutanées dues au vieillissement, présentes sur le visage et/ou le corps, y compris les mains et le cuir chevelu ainsi que pour la préparation d'une crème destinée au traitement des taches de la peau d'origine pathologique.

L'invention se rapporte aussi à l'utilisation de la composition ci-dessus pour protéger la peau contre les radicaux libres et/ou les rayons solaires.

Par radicaux libres, il faut entendre toutes les espèces radicalaires et/ou ioniques de l'oxygène et des composés oxygénés, y compris l'oxygène singulet.

L'invention se rapporte encore à l'utilisation de l'arbutine comme actif antiradicaux libres, dans une composition cosmétique et/ou dermatologique.

L'invention a encore pour objet un procédé de traitement cosmétique et/ou dermatologique de la peau, consistant à appliquer la composition définie ci-dessus sur la peau.

D'autres caractéristiques et avantages de l'invention ressortiront mieux de la description qui suit, donnée à titre illustratif et non limitatif. Dans les exemples ci-après les compositions sont données en % pondéral.

Les tests suivants permettent de mettre en évidence les avantages de la présente invention.

### Mise en évidence de l'activité de protection solaire de l'association d'arbutine et d'un dérivé sulfonaté de benzylidène camphre

Le but du test est de montrer l'aptitude de la composition de l'invention à protéger la peau contre les UVA grâce à un indice de protection UVA élevé.

Les sujets testés sont au nombre de dix. Ils sont des deux sexes, adultes et sains. Une zone cutanée située sur la peau du dos de chacun des volontaires est soumise à une exposition solaire après définition du type de peau. L'exposition est effectuée avec un spectre UVA seul (320 nm à 400 nm), fourni par une lampe au xénon de 150 W munie des filtres WG 335 de 3 mm d'épaisseur et UG 11 de 1 mm d'épaisseur. Le rayonnement UV est conduit par 6 guides de lumière liquides et flexibles, permettant de délivrer des doses de 6 à 45 Joules/cm² en progression géométrique de 50 %.

L'application se fait en usage unique, à l'air libre de 2 ± 0,02 µl/cm², soit 0,15 ml environ par sujet de produit à étudier, sur une surface de 75 cm², mesuré avec une seringue stérile de 1ml Turberculine, graduée au 1/1000^{e} et à cône centré.

L'exposition se fait 15 min environ après l'application du produit. L'examen macroscopique cutané des zones exposées est réalisé 2 h environ après les expositions de manière à évaluer les réactions de pigmentation immédiate et permanente. La dose Pigmentante Minimale sur la zone protégée par le produit appliqué (DPM protégée) et celle sur la zone où aucune application n'a été appliquée (DPM non protégée) sont réalisées simultanément.

L'indice de protection IP est ensuite calculé de la manière suivante :
IP=DPM protégée/DPM non protégée.

On a appliqué trois compositions ayant le même support et contenant respectivement de l'arbutine, le filtre (acide téréphtalylidène di-camphosulfonique) et l'association d'arbutine et le filtre, et on a déterminé les indices de protection UVA de ces compositions.

Les indices de protection (IP) sont indiqués dans le tableau suivant :

| Composition | Indice de protection UVA (IP) |
|---|---|
| avec 5 % d'arbutine | 1,6 |
| avec 1 % d'acide téréphtalylidène di-camphosulfonique | 2,7 |
| avec 5 % d'arbutine et 1 % d'acide téréphtalylidène di-camphosulfonique | 3,4 |

Ces résultats montrent une potentialisation significative de l'indice de protection du filtre par l'arbutine.

### Mise en évidence de la synergie d'activité antiradicalaire (ARL) de l'arbutine et d'un dérivé sulfonaté de benzylidène camphre

L'activité ARL a été mise en évidence par un test d'inhibition de la production d'éthylène :

Dans une boîte de Pétri de diamètre 32 mm, sont introduits dans l'ordre suivant :
- 1,4 ml de tampon phosphate 50 mM (pH = 7,4),
- 100 µl de solution 200 mM de méthionine,
- 100 µl de solution 4 mM de chlorure ferrique,
- 100 µl du produit à tester,
- 100 µl de solution 4 mM d'E.D.T.A.(acide éthylènediamine tétracétique),
- 100 µl de solution 400 mM de N.A.D.H. (nicotinamide adénine dinucléotide, forme réduite),
- 100 µl de solution 2 mM de riboflavine.

Le mélange a un volume total de 2 ml.

La boîte de Pétri est ensuite placée sur une coupelle en aluminium et recouverte d'une cellule en quartz afin d'être exposée sous les U.V.A. (365 nm) à la dose égale à 1 Joule/cm2. L'ensemble composé de N.A.D.H., de riboflavine, de chlorure ferrique et d'E.D.T.A., soumis à l'exposition des U.V.A., va générer des espèces réduites de l'oxygène : O_{2°}⁻, H₂O₂, et principalement le radical hydroxyle °OH. Ce dernier va réagir avec la méthionine en libérant de l'éthylène dont la quantité est mesurée par chromatographie en phase gazeuse.

Plus la quantité de radicaux libres présents est petite, plus est faible la quantité d'éthyléne libéré. Les résultats sont exprimés en pourcentage de pouvoir inhibiteur correspondant au pourcentage de diminution de la production d'éthylène par rapport au témoin (contenant 100 µl de tampon phosphate remplaçant le produit à tester).

Les substances étudiées sont introduites à l'aide d'une micropipette. Ce sont :
- l'arbutine
- l'acide téréphtalylidène di-camphosulfonique

Conditions chromatographiques : (appareil de type VARIAN 3740)
- température injecteur : 80°C,
- température colonne : 80°C,
- température détecteur : 250°C,
- pression d'hélium : 36 psi (soit environ 2,4.105 Pa),
- colonne : alumina F1 60/80 mesh (origine : Supelco),
- longueur : 2 m,
- diamètre externe : 1/8.

Les résultats (moyenne de 3 essais) sont résumés dans le tableau suivant :

| Echantillon | % d'inhibition |
|---|---|
| Arbutine à 0,2 % | 39,0 ± 0,6 |
| Acide téréphtalylidène dicamphosulfonique à 0,2% | 44,7 ± 1,7 |
| Arbutine à 0,1 % + Acide téréphtalylidène di-campho-sulfonique à 0,1 % | 52,8 ± 1,5 |

Ces résultats montrent la synergie d'activité ARL de l'association selon l'invention.

### EXEMPLE 1 : Crème eau-dans-huile pour la protection de la peau contre les rayons UVA et/ou prévention de la pigmentation de la peau

### Composition

| | | |
|---|---|---|
| *A*_{*1*} | - Tri-stéarate de Sorbitane (émulsionnant) | 0,9 % |
| | - Stéarate de polyéthylène glycol (40.OE) (émulsionnant). | 2,0 % |
| | - Alcool cétylique d'origine naturelle (co-émulsionnant) | 4,0 % |
| | - Mono,di,tri-palmitostéarate de glycéryle (émulsionnant). | 3,0 % |
| | - Myristate de myristyle (huile) | 2,0 % |
| | - Palmitate d'éthyl-2 hexyle (huile) | 5,0 % |
| | - Iso-paraffine (6-8 moles d'isobutylène) hydrogénée (huile) | 6,5 % |
| *A*_{*2*} | - Cyclopentadiméthylsiloxane (huile) | 5,0 % |
| *B* | - Eau déminéralisée stérilisée | qsp 100 % |
| | - Glycérol (hydratant) | 3,0 % |
| | - Arbutine | 5,0 % |
| | - Alcool éthylique absolu dénaturé | 10,0 % |
| | - Para-hydroxybenzoate de méthyle (conservateur) | 0,2 % |
| *C* | - Acide téréphtalylidène di-camphosulfonique dans l'eau à 33 % | 3,33 % |
| | - Triéthanolamine (neutralisant) | 0,67 % |

### Préparation de la phase A₁ + A₂

Les constituants de A₁ sont solubilisés à 80 °C. Lorsque le mélange est limpide, la température est abaissée à 65 °C et on ajoute A₂. Le mélange doit être limpide et homogène. On maintient la température de 65 °C.

### Fabrication

Dans un bécher de fabrication, les constituants de B sont solubilisés à 85 °C-90 °C. Après vérification de la limpidité, la température est ramenée à 65 °C. On réalise l'émulsion, sous agitation, en versant (A₁+A₂) dans B. On poursuit le refroidissement sous agitation. A 40 °C on ajoute la phase C, toujours sous agitation, et on laisse refroidir à 20 °C sous agitation.

Les compositions 2, 3 et 4 se préparent de la même façon.

### EXEMPLE 2 : Crème huile-dans-eau anti-taches

### Composition

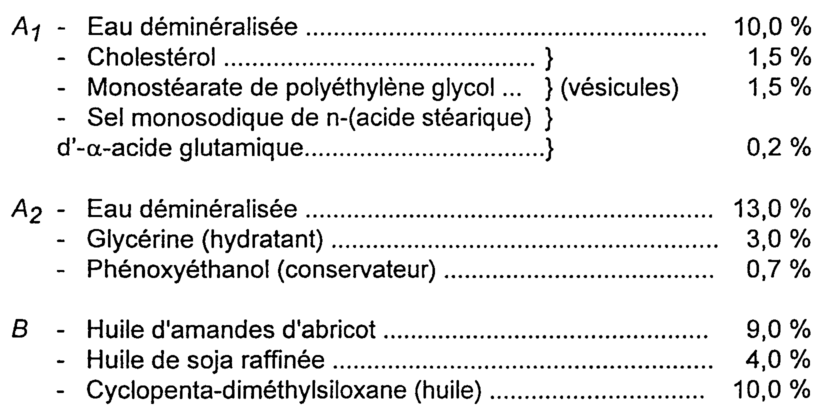

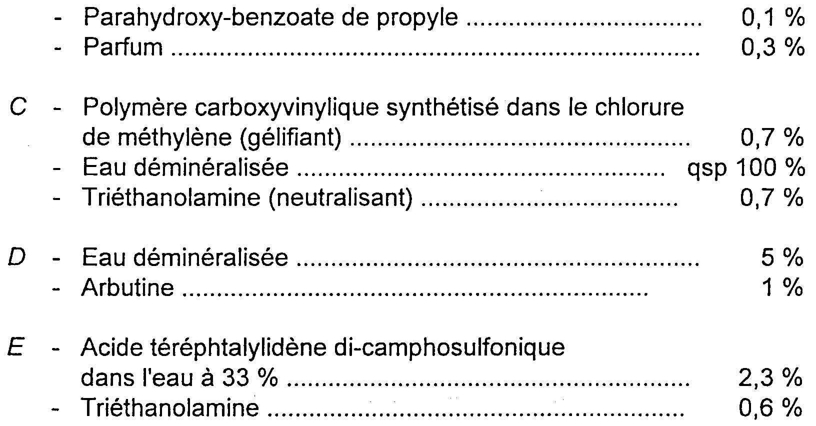

### Fabrication

On fait fondre les constituants de A₁ à 100 °C. On laisse gonfler sous agitation pendant 1H30. Lorsque le mélange est homogène on ajoute A₂ ; on stabilise la température à 80 ⁰C. On effectue alors deux passages à l'homogénéisateur haute pression pour former les vésicules. On prépare B à 70 °C ; le mélange doit être limpide. On refroidit à 50 °C. On ajoute B dans A à 50 °C.

On fait alors deux passages à l'homogénéisateur haute pression pour disperser la phase grasse B. On refroidit à 30 °C. On ajoute C (le gel aura été préalablement préparé dans l'eau à 80 °C, en saupoudrant le polymère carboxyvinylique ; après gonflement de ce dernier, on neutralise à la triéthanolamine sous agitation ; le gel doit être bien lisse). On ajoute D puis E. On maintient l'agitation pendant 5 minutes. La fabrication est terminée.

### EXEMPLE 3 : Gel protecteur contre les rayons solaires

### Composition

| | | |
|---|---|---|
| *A* | - Eau déminéralisée | qsp 100 % |
| | - Polymère carboxyvinylique | 0,45 % |
| | - Triéthanolamine | 0,45 % |
| *B* | - Eau déminéralisée | 59,8 % |
| | - Glycérine | 3 % |
| | - Para-hydroxybenzoate de méthyle | 0,2 % |
| | - Arbutine | 1 % |
| | - Gomme de Xanthane (gélifiant) | 0,2 % |
| *C* | - Acide téréphtalylidène di-camphosulfonique dans l'eau à 33 % | 2,3 % |
| | - Triéthanolamine | 0,6 % |

### Fabrication

Dans l'eau à 80 °C, on prépare le gel (polymère carboxyvinylique) comme dans l'exemple 2. Après solubilisation des constituants de B à 80 °C, on ajoute B dans A. On lisse et on laisse refroidir sous agitation à pales lentes. A 35 °C, on ajoute C. On laisse refroidir à 25 °C. La fabrication du gel est terminée.

### EXEMPLE 4 : Lotion 〈〈teint clair〉〉

### Composition

| | | |
|---|---|---|
| *A* | - Triglycérides ricinoléiques hydrogénés oxyéthylénés (60 OE) (peptisant) | 0,09 % |
| | - Parfum | 0,03 % |
| *B* | - Eau déminéralisée | qsp 100 % |
| | - Glycérine | 5,5 % |
| | - Arbutine | 1 % |
| | - Acide citrique | 1 % |
| | - Triéthanolamine à 99 % | 1,9 % |
| | - Imidazolidinyl urée (conservateur) | 0,3 % |
| *C* | - Acide téréphtalylidène di-camphosulfonique dans l'eau à 33 % | 2,3 % |
| | - Triéthanolamine | 0,6 % |

### Fabrication

On mélange les constituants de A à 40 °C. Lorsqu'ils sont parfaitement solubilisés, on ajoute successivement les constituants de B à température ambiante. On maintient l'agitation et on vérifie la bonne solubilisation des constituants. On ajoute C ; le mélange doit être limpide. La fabrication est terminée.

## Revendications

1. Composition pour application topique, caractérisée en ce qu'elle contient dans un milieu cosmétiquement et/ou dermatologiquement acceptable, de l'arbutine et au moins un filtre ultraviolet choisi dans le groupe constitué du benzylidène camphre et de ses dérivés, l'arbutine et le filtre étant en une quantité telle que cette association présente une synergie d'activité contre les radicaux libres.

2. Composition selon la revendication 1, caractérisée en ce que le filtre est un dérivé sulfoné ou sulfonaté du benzylidène camphre.

3. Composition pour application topique, caractérisée en ce qu'elle contient dans un milieu cosmétiquement et/ou dermatologiquement acceptable, de l'arbutine et au moins un filtre ultraviolet choisi dans le groupe constitué des dérivés sulfonés ou sulfonatés du benzylidène camphre.

4. Composition, selon l'une quelconque des revendications précédentes , caractérisée en ce que le dérivé du benzylidène camphre présente la formule (I) suivante : 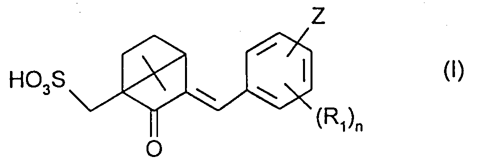 dans laquelle :
- Z désigne un groupement 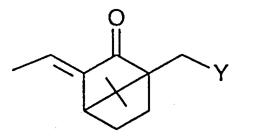 dans lequel Y représente -H ou -SO₃H, éventuellement neutralisé,
- n est égal à 0 ou est un nombre allant de 1 à 4 (0 ≤n≤ 4),
- R₁, représente un ou plusieurs radicaux alkyle ou alkoxy, identiques ou différents, linéaires ou ramifiés, contenant de 1 à 4 atomes de carbone.

5. Composition selon l'une quelconque des revendications précédentes, caractérisée en ce que le dérivé du benzylidène camphre est l'acide benzène 1,4 [di(3-méthylidène campho-10-sulfonique)].

6. Composition selon l'une quelconque des revendications précédentes, caractérisée en ce qu'elle se présente sous forme d'une émulsion, d'un gel ou sous forme d'une dispersion de sphérules.

7. Composition selon l'une quelconque des revendications précédentes, caractérisée en ce que l'arbutine représente de 0,05 % à 10 % en poids par rapport au poids total de la composition.

8. Composition selon l'une quelconque des revendications précédentes, caractérisée en ce que ledit filtre ultraviolet représente de 0,1 % à 10 % en poids par rapport au poids total de la composition.

9. Composition selon l'une quelconque des revendications précédentes, caractérisée en ce qu'elle contient 0,05 à 0,15 % d'arbutine et 0,05 à 0,15 % de filtre ultraviolet.

10. Composition selon l'une quelconque des revendications précédentes, caractérisée en ce qu'elle contient aussi des adjuvants hydrophiles ou lipophiles.

11. Composition selon l'une quelconque des revendications précédentes, caractérisée en ce que les adjuvants sont choisis parmi les gélifiants, les conservateurs, les parfums, les charges, les matières colorantes, les actifs autres que l'arbutine et ledit filtre ultraviolet.

12. Utilisation cosmétique de la composition selon l'une quelconque des revendications précédentes pour protéger la peau contre les radicaux libres et/ou les rayons solaires.

13. Utilisation cosmétique de la composition selon l'une quelconque des revendications 1 à 11, pour dépigmenter la peau et/ou traiter les taches cutanées dues au vieillissement.

14. Utilisation cosmétique de la composition selon l'une quelconque des revendications 1 à 11, pour traiter les rides et/ou les ridules de la peau.

15. Utilisation cosmétique de la composition selon l'une quelconque des revendications 1 à 11, pour tonifier, protéger, et/ou raffermir la peau.

16. Utilisation de la composition selon l'une quelconque des revendications 1 à 11, pour préparer une crème destinée au traitement des maladies de peau conférant à cette dernière des taches.

17. Procédé de traitement cosmétique de la peau, caractérisé en ce qu'il consiste à appliquer sur la peau une composition selon l'une quelconque des revendications 1 à 11.

18. Utilisation de l'arbutine comme actif antiradicaux libres, dans une composition cosmétique et/ou dermatologique.

## Claims

1. Composition for topical application, characterized in that it contains, in a cosmetically and/or dermatologically acceptable medium, arbutin and at least one ultraviolet screening agent chosen from the group consisting of benzylidenecamphor and derivatives thereof, the arbutin and the screening agent being in an amount such that this combination displays a synergism of activity against free radicals.

2. Composition according to Claim 1, characterized in that the screening agent is a sulphone-containing or sulphonate-containing derivative of benzylidenecamphor.

3. Composition for topical application, characterized in that it contains, in a cosmetically and/or dermatologically acceptable medium, arbutin and at least one ultraviolet screening agent chosen from the group consisting of sulphone-containing or sulphonate-containing derivatives of benzylidenecamphor.

4. Composition according to any one of the preceding claims, characterized in that the benzylidenecamphor derivative has the following formula (I): 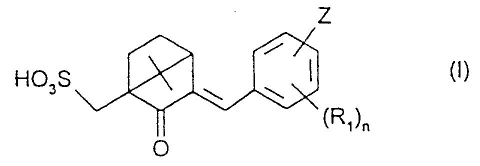 in which:
- Z denotes a group 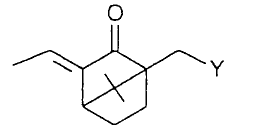 in which Y represents -H or -SO₃H, which is optionally neutralized,
- n is equal to 0 or is a number ranging from 1 to 4 (0 ≤ n ≤ 4),
- R₁ represents one or more linear or branched alkyl or alkoxy radicals, which may be identical or different, containing from 1 to 4 carbon atoms.

5. Composition according to any one of the preceding claims, characterized in that the benzylidenecamphor derivative is benzene-1,4-bis(3-methylidenecamphor-10-sulphonic acid).

6. Composition according to any one of the preceding claims, characterized in that it is in the form of an emulsion or a gel or in the form of a dispersion of spherules.

7. Composition according to any one of the preceding claims, characterized in that the arbutin represents from 0.05 % to 10 % by weight relative to the total weight of the composition.

8. Composition according to any one of the preceding claims, characterized in that the said ultraviolet screening agent represents from 0.1 % to 10 % by weight relative to the total weight of the composition.

9. Composition according to any one of the preceding claims, characterized in that it contains 0.05 to 0.15% arbutin and 0.05 to 0.15% ultraviolet screening agent.

10. Composition according to any one of the preceding claims, characterized in that it also contains hydrophilic or lipophilic adjuvants.

11. Composition according to any one of the preceding claims, characterized in that the adjuvants are chosen from gelling agents, preserving agents, fragrances, fillers, dyestuffs and active agents other than arbutin and the said ultraviolet screening agent.

12. Cosmetic use of the composition according to any one of the preceding claims in order to protect the skin against free radicals and/or solar rays.

13. Cosmetic use of the composition according to any one of Claims 1 to 11 in order to depigment the skin and/or to treat skin blemishes due to ageing.

14. Cosmetic use of the composition according to any one of Claims 1 to 11 in order to treat wrinkles and/or fine lines on the skin.

15. Use of the composition according to any one of Claims 1 to 11 in order to tone, protect and/or firm up the skin.

16. Use of the composition according to any one of Claims 1 to 11 in order to prepare a cream intended for the treatment of skin diseases which leave blemishes on it.

17. Process for the cosmetic treatment of the skin, characterized in that it consists in applying a composition according to any one of Claims 1 to 11 to the skin.

18. Use of arbutin as an anti-free-radical active agent in a cosmetic and/or dermatological composition.

## Patentansprüche

1. Zusammensetzung zur topischen Anwendung,
dadurch gekennzeichnet, daß
sie in einem kosmetisch und/oder dermatologisch akzeptablen Medium Arbutin und mindestens ein UV-Filter enthält, der unter Benzylidencampher und seinen Derivaten ausgewählt ist, wobei das Arbutin und das Filter in einem solchen Mengenanteil vorliegen, das die Kombination eine synergistische Wirksamkeit gegen freie Radikale aufweist.

2. Zusammensetzung nach Anspruch 1, dadurch gekennzeichnet, daß das Filter ein Sulfonsäurederivat oder Sulfonatderivat von Benzylidencampher ist.

3. Zusammensetzung zur topischen Anwendung, dadurch gekennzeichnet, daß sie in einem kosmetisch und/oder dermatologisch akzeptablen Medium Arbutin und mindestens ein UV-Filter enthält, das unter den Sulfonsäurederivaten oder Sulfonatderivaten von Benzylidencampher ausgewählt ist.

4. Zusammensetzung nach einem der vorhergehenden Ansprüche, dadurch gekennzeichnet, daß das Benzylidencampherderivat die folgende Formel (I) aufweist 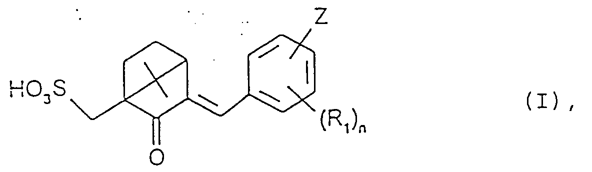 worin bedeuten:
- Z eine Gruppe 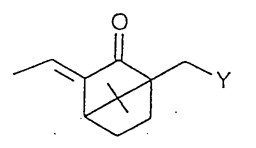 worin Y -H oder -SO₃H, das gegebenenfalls neutralisiert ist, bedeutet,
- n Null oder eine Zahl im Bereich von 1 bis 4 (0 ≤ n ≤ 4),
- R₁ eine oder mehrere geradkettige oder verzweigte Alkyl- oder Alkoxygruppen mit 1 bis 4 Kohlenstoffatomen, die gleich oder voneinander verschieden sind.

5. Zusammensetzung nach einem der vorhergehenden Ansprüche, dadurch gekennzeichnet, daß das Benzylidencampherderivat Benzol-1,4-[di(3-methylidencampher-10-sulfonsäure)] ist.

6. Zusammensetzung nach einem der vorhergehenden Ansprüche, dadurch gekennzeichnet, daß sie in Form einer Emulsion, als Gel oder in Form einer Dispersion von Kügelchen vorliegt.

7. Zusammensetzung nach einem der vorhergehenden Anspruche, dadurch gekennzeichnet, daß das Arbutin 0,05 bis 10 Gew.-%, bezogen auf das Gesamtgewicht der Zusammensetzung, ausmacht.

8. Zusammensetzung nach einem der vorhergehenden Ansprüche, dadurch gekennzeichnet, daß das UV-Filter 0,1 bis 10 Gew.-%, bezogen auf das Gesamtgewicht der Zusammensetzung, ausmacht.

9. Zusammensetzung nach einem der vorhergehenden Ansprüche, dadurch gekennzeichnet, daß sie 0,05 bis 0,15 % Arbutin und 0,05 bis 0,15 % UV-Filter enthält.

10. Zusammensetzung nach einem der vorhergehenden Ansprüche, dadurch gekennzeichnet, daß sie ferner hydrophile oder lipophile Hilfsstoffe enthält.

11. Zusammensetzung nach einem der vorhergehenden Ansprüche, dadurch gekennzeichnet, daß die Hilfsstoffe unter den Gelbildnern, Konservierungsmitteln, Parfums, Füllstoffen, Färbemitteln und Wirkstoffen, die von Arbutin und dem UV-Filter verschieden sind, ausgewählt sind.

12. Kosmetische Verwendung der Zusammensetzung nach einem der vorhergehenden Ansprüche zum Schutz der Haut gegen freie Radikale und/oder Sonnenlicht.

13. Kosmetische Verwendung der Zusammensetzung nach einem der Ansprüche 1 bis 11 zur Depigmentierung der Haut und/oder Behandlung von altersbedingten Hautflecken.

14. Kosmetische Verwendung der Zusammensetzung nach einem der Ansprüche 1 bis 11 zur Behandlung von Falten und/oder Fältchen der Haut.

15. Kosmetische Verwendung der Zusammensetzung nach einem der Ansprüche 1 bis 11 zur Belebung, zum Schutz und/oder zur Straffung der Haut.

16. Verwendung der Zusammensetzung nach einem der Ansprüche 1 bis 11 zur Herstellung einer Creme, die zur Behandlung von Hautkrankheiten bestimmt ist, welche auf der Haut Flecken hervorrufen.

17. Verfahren zur kosmetischen Behandlung der Haut, dadurch gekennzeichnet, daß es darin besteht, auf die Haut eine Zusammensetzung nach einem der Ansprüche 1 bis 11 aufzutragen.

18. Verwendung von Arbutin als Wirkstoff gegen freie Radikale in einer kosmetischen und/oder dermatologischen Zusammensetzung.
